# EUROPÄISCHE PATENTANMELDUNG

(11) **EP 4 488 250 A1**
(43) Veröffentlichungstag der Anmeldung: **08.01.2025**
(21) Anmeldenummer: 23183865.7
(22) Anmeldetag: 06.07.2023
(51) Int. Cl.: C07C 1/20, C07C 7/00, C07C 7/04, C07C 7/09, C07C 11/02, C07C 11/04, C07C 11/06, C07C 15/02

(54) **VERFAHREN ZUR HERSTELLUNG VON OLEFINEN UND BTX-AROMATEN AUS OXYGENATEN**

(71) Anmelder: L'AIR LIQUIDE, SOCIETE ANONYME POUR L'ETUDE ET L'EXPLOITATION DES PROCEDES GEORGES CLAUDE, 75007 Paris (FR)
(72) Erfinder: Renner, Thomas, 60388 Frankfurt am Main (DE); Lin, Lin, 60388 Frankfurt am Main (DE); Haag, Stephane, 60388 Frankfurt am Main (DE); Williams, Bryce, 60388 Frankfurt (DE)
(74) Vertreter: Air Liquide

(57) **Zusammenfassung**

Die Erfindung betrifft ein Verfahren und eine Anlage zur Herstellung von Olefinen aus Oxygenaten (OTO), wobei gleichzeitig auch die Ausbeute der BTX-Aromaten Benzol, Toluol und der isomeren Xylole optimiert werden soll. Erfindungsgemäß wird dazu die bei dem OTO-Verfahren gemäß Stand der Technik erhaltene Benzinfraktion einem Aromatisierungsreaktor zugegeben. Durch mehrstufige Auftrennung des Produktstroms des Aromatisierungsreaktors wird ein BTX-Aromaten erhaltender Produktstrom und eine an Olefinen, Cycloalkanen und Cycloalkenen abgereicherte Benzinfraktion erhalten.

## Beschreibung

### Gebiet der Erfindung

Die Erfindung betrifft ein Verfahren und eine Anlage zur Herstellung von Olefinen aus Oxygenaten (OTO), wobei gleichzeitig auch die Ausbeute der BTX-Aromaten Benzol, Toluol und der isomeren Xylole optimiert werden soll.

### Stand der Technik

Kurzkettige Olefine, beispielsweise Propylen (Propen) und Ethylen (Ethen), gehören zu den wichtigsten Grundstoffen der chemischen Industrie. Dies liegt darin begründet, das ausgehend von diesen ungesättigten Verbindungen mit einer kurzen Kettenlänge Moleküle mit langkettigem Kohlenstoffgerüst und zusätzlichen Funktionalisierungen aufgebaut werden können. Insbesondere bei der Erzeugung von Kunststoffen durch Polymerisation finden diese kurzkettigen Olefine weite Anwendung.

Als Quelle für kurzkettige Olefine diente in der Vergangenheit vor allem das Steam-Cracking, d. h. die thermische Spaltung bei der Erdölverarbeitung. In den vergangenen Jahren wurden jedoch weitere Prozesse zur Herstellung von kurzkettigen Olefinen entwickelt. Dies ist zum einen durch steigende Nachfrage bedingt, die durch die vorhandenen Quellen nicht mehr gedeckt werden kann; zum anderen fordert die zunehmende Verknappung von fossilen Rohstoffen die Verwendung anderer Ausgangsstoffe.

Die sog. MTP- (Methanol-to-Propylen) oder auch MTO- (Methanol-to-Olefin) Verfahren zur Herstellung von Propylen und anderen kurzkettigen Olefinen gehen von Methanol als Ausgangsstoff aus. Verallgemeinernd spricht man in diesem Zusammenhang auch von Oxygenat-zu-Olefin-(OTO)-Verfahren, da sauerstoffhaltige organische Komponenten wie Methanol oder Dimethylether (DME) auch als Oxygenate bezeichnet werden. In diesen heterogen katalysierten Verfahren wird demnach beispielsweise aus Methanol zunächst teilweise das Zwischenprodukt Dimethylether und nachfolgend aus einer Mischung von Methanol und Dimethylether eine Mischung aus Ethylen und Propylen sowie Kohlenwasserstoffen mit höherer Molmasse, darunter auch Olefine, gebildet. Zudem findet sich im Produktstrom Wasser, welches einerseits einen aus dem Prozessdampf stammt, der optional dem MTO-Reaktor zur Reaktionsmodulierung zugeführt wird und andererseits aus dem im MTP-Reaktor erzeugten Reaktionswasser, das als Koppelprodukt der Olefinbildungsreaktion entsteht.

Die sich anschließende Aufreinigung soll zum einen unerwünschte Nebenprodukte und unumgesetzte Edukte abtrennen und die einzelnen Kohlenwasserstofffraktionen möglichst rein darstellen. Üblicherweise wird hierzu im ersten Schritt ein Quenchsystem zur Anwendung gebracht. Unter Quenchen wird dabei eine schlagartige oder schockartige Abkühlung verstanden, die zumeist durch direkten Wärmeaustausch mit einem fluiden Quenchmedium bewirkt wird. Wenn hierzu eine Flüssigkeit wie Wasser oder Methanol verwendet wird, tritt zudem eine gewisse Reinigungswirkung in Bezug auf die verbliebene Gasphase auf.

Ein Beispiel für die einer OTO-Reaktion nachfolgende Aufreinigung findet sich in der Patentveröffentlichung DE 10 2014 112 792 A1, in der beschrieben wird, wie in einem ersten Schritt eine heterogen-katalysierte Umsetzung von wenigstens einem Oxygenat zu einem C2 Olefine, C3 Olefine, C4 Olefine, C5/6 Kohlenwasserstoffverbindungen und C7+ Kohlenwasserstoffverbindungen enthaltenden Produktstrom und in einem zweiten Schritt eine Abtrennung eines zu wenigstens 95 Gew.-% aus C3 Olefinen bestehenden Propylenstroms erzeugt wird.

Die weiteren Aufreinigungseinheiten, die in der DE 10 2014 112 792 A1 beschrieben werden, entsprechen dem im Stand der Technik üblichen Konzept. Durch das Quenchen kann bereits eine grobe Trennung der Fraktionen in Abhängigkeit von ihrer Kettenlänge der entstehenden Olefine aufgrund des teilweisen Auskondensierens erfolgen, so dass eine flüssige C4+ Fraktion aus dem Quench abgeführt werden kann. Die gasförmig abgetrennte C4- Fraktion wird anschließend in eine Kompressionsstufe eingespeist. Die aus der Kompression stammende C4- Fraktion wird dann einer Trennvorrichtung zugeführt, in der C3- Kohlenwasserstoffe von den C4+ Kohlenwasserstoffen separiert werden. In anschließenden Reinigungsschritten wird die C3 Fraktion in einer weiteren Trenneinrichtung von der C2- Fraktion getrennt, was aufgrund der geringen Siedepunkte der beiden Fraktionen unter Druck erfolgen muss. Insgesamt ist die Aufreinigung des Produktstroms kompliziert, da eine hohe Reinheit der Produkte angestrebt wird. Dies gilt für MTP-Anlagen mit üblichen Produktionskapazitäten von etwa 470 kta Propylen, wird aber noch entscheidender, wenn die Anlagenkapazität geringer ist (100 kta oder 200 kta Propylen). Dadurch sind kleine Anlagen wirtschaftlich weniger rentabel.

Insgesamt umfassen die Produkte des MTP-Verfahrens einen Propylenproduktstrom, einen Ethylenproduktstrom und eine Benzinfraktion, wobei letztere höhere Paraffine, Olefine, Aromaten und cyclische Kohlenwasserstoffe enthält. Ferner werden innerhalb des Verfahrens verschiedene Kohlenwasserstoffe, vor allem C4 Olefine, C5 Olefine und C6+ Olefine enthaltende Ströme gewonnen, die überwiegend oder vollständig zum MTP-Reaktor zurückgeführt werden und dort katalytisch zu kurzkettigen Olefinen gespaltet werden, was die Ausbeute der Zielprodukte Propylen und Ethylen verbessert.

Das MTP-Verfahren zielt in seiner bisherigen Ausgestaltung vor allem auf die Maximierung der Ausbeute an Propylen ab, das ein besonders begehrtes Produkt darstellt. Es besteht allerdings auch das Bedürfnis, die Ausbeute an anderen Wertstoffen, beispielsweise an aromatischen Kohlenwasserstoffen, insbesondere an BTX-Aromaten (Benzol, Toluol, Xylole) zu erhöhen, ohne die Ausbeute der primären Zielprodukte Propylen und Ethylen wesentlich zu verringern.

Hierfür bieten die aus dem Stand der Technik bekannten Ausgestaltungen des MTP-Verfahrens und anderer OTO-Verfahren bislang keine befriedigende Lösung an.

### Beschreibung der Erfindung

Es ist daher die Aufgabe der vorliegenden Erfindung, ein Verfahren und eine Anlage zur Herstellung von kurzkettigen Olefinen aus Oxygenaten, insbesondere Ethylen und Propylen, mit zusätzlicher Produktion von BTX-Aromaten vorzuschlagen, die die genannten Nachteile von OTO-Verfahren vermeiden, die bislang aus dem Stand der Technik bekannt sind. Diese Aufgabe wird in einem ersten Aspekt der Erfindung durch ein Verfahren mit den Merkmalen des Anspruchs 1 und in einem weiteren Aspekt der Erfindung durch eine Anlage mit den Merkmalen des Anspruchs 10 gelöst. Weitere Aspekte ergeben sich aus den abhängigen Verfahrensansprüchen.

Die für die Umsetzung von Oxygenaten zu Olefinprodukten erforderlichen Oxygenatumwandlungsbedingungen sind dem Fachmann aus dem Stand der Technik, beispielsweise den eingangs erörterten Druckschriften, bekannt. Es sind diejenigen physikalisch-chemischen Bedingungen, unter denen ein messbarer, bevorzugt ein technisch relevanter Umsatz von Oxygenaten zu Olefinen erzielt wird. Notwendige Anpassungen dieser Bedingungen an die jeweiligen Betriebserfordernisse wird der Fachmann auf der Grundlage von Routineversuchen vornehmen. Dabei können ggf. offenbarte, spezifische Reaktionsbedingungen als Orientierung dienen, sie sind aber in Bezug auf den Umfang der Erfindung nicht einschränkend zu verstehen.

Thermische Trennverfahren im Sinne der vorliegenden Erfindung sind alle Trennverfahren, die auf der Einstellung eines thermodynamischen Phasengleichgewichtes beruhen. Bevorzugt sind dies die Destillation oder Rektifikation. Grundsätzlich ist aber auch der Einsatz anderer thermischer Trennverfahren denkbar, beispielsweise der Extraktion oder Extraktivdestillation.

Wenn angegeben wird, dass ein Strom Kohlenwasserstoffe umfasst und spezifisch Olefine umfasst, ist dies so zu verstehen, dass die Olefine eine spezifische Untergruppe der Kohlenwasserstoffe darstellen und dass demnach auch andere, nicht-olefinische Kohlenwasserstoffe in dem Strom enthalten sein können.

Unter Oxygenaten werden grundsätzlich alle sauerstoffhaltigen Kohlenwasserstoffverbindungen verstanden, die sich unter Oxygenatumsetzungsbedingungen zu Olefinen, insbesondere zu kurzkettigen Olefinen wie Propylen, und weiteren Kohlenwasserstoffprodukten umsetzen lassen. Beispiele sind Methanol oder Dimethylether (DME).

Als kurzkettige Olefine werden im Rahmen der vorliegenden Erfindung insbesondere die Olefine verstanden, die bei Umgebungsbedingungen gasförmig vorliegen, beispielsweise Ethylen, Propylen sowie die isomeren Butene 1-Buten, cis-2-Buten, trans-2-Buten, iso-Buten.

Als höhere Kohlenwasserstoffe oder längerkettige Kohlenwasserstoffe werden im Rahmen der vorliegenden Erfindung insbesondere diejenigen Kohlenwasserstoffe verstanden, die bei Umgebungsbedingungen in Reinform flüssig vorliegen.

Zur Bezeichnung von Kohlenwasserstofffraktionen wird folgende Nomenklatur verwendet: "Cn Fraktion" bezeichnet eine Kohlenwasserstofffraktion, die überwiegend Kohlenwasserstoffe der C-Kettenlänge n, also mit n C-Atomen, enthält. "Cn-Fraktion" bezeichnet eine Kohlenwasserstofffraktion, die überwiegend Kohlenwasserstoffe der C-Kettenlänge n sowie mit niedrigeren C-Kettenlängen enthält. "Cn+ Fraktion" bezeichnet eine Kohlenwasserstofffraktion, die überwiegend Kohlenwasserstoffe der C-Kettenlänge n sowie mit höheren C-Kettenlängen enthält. Aufgrund der verwendeten physikalischen Trennverfahren, beispielsweise der Destillation, ist die Auftrennung hinsichtlich der C-Kettenlänge nicht in der Weise zu verstehen, dass Kohlenwasserstoffe mit anderer Kettenlänge rigoros ausgeschlossen werden. So enthält eine Cn- Fraktion je nach Verfahrensbedingungen des Trennverfahrens immer noch geringe Mengen von Kohlenwasserstoffen mit einer C-Zahl größer als n.

Die genannten Aggregatzustände fest, flüssig und gasförmig bzw. dampfförmig sind immer in Bezug auf die lokalen physikalischen Bedingungen zu verstehen, die bei dem jeweiligen Verfahrensschritt oder in dem jeweiligen Anlagenteil herrschen, sofern nichts anderes angegeben ist. Im Rahmen der vorliegende Anmeldung sind die Aggregatzustände gasförmig bzw. dampfförmig als synonym zu betrachten.

Unter einem Aufteilen oder Auftrennen bzw. Abtrennen eines Stoffstroms ist im Zusammenhang mit der vorliegenden Erfindung die Erzeugung mindestens zweier Teilströme aus dem ursprünglichen Stoffstrom zu verstehen, wobei mit dem Auftrennen bzw. Abtrennen eine beabsichtigte Änderung der stofflichen Zusammensetzung der erhaltenen Teilströme in Bezug auf den ursprünglichen Stoffstrom verbunden ist, beispielsweise durch Anwendung eines thermischen Trennverfahrens oder zumindest thermischen Trennschrittes auf den ursprünglichen Stoffstrom. Dagegen ist mit dem Aufteilen des ursprünglichen Stoffstroms in der Regel keine Änderung der stofflichen Zusammensetzung der erhaltenen Teilströme verbunden.

Unter einer Benzinfraktion ist ein überwiegend, bevorzugt weitgehend vollständig aus höheren Kohlenwasserstoffen bestehendes, unter Umgebungsbedingungen flüssig vorliegendes Stoffgemisch zu verstehen, das geeignet sein kann, als Ottokraftstoff verwendet zu werden.

Unter dem überwiegenden Teil einer Fraktion, eines Stoffstroms etc. ist ein Anteil zu verstehen, der mengenmäßig größer ist als alle anderen jeweils für sich betrachteten Anteile. Insbesondere bei binären Gemischen oder bei der Auftrennung einer Fraktion in zwei Teile ist darunter ein Anteil von mehr als 50 Gew.-% zu verstehen, sofern im konkreten Fall nichts anderes angegeben ist.

Unter der Angabe, dass ein Stoffstrom überwiegend aus einer Komponente oder Komponentengruppe besteht, ist zu verstehen, dass der Stoffmengenanteil (Molenbruch) oder Massenanteil (Massenbruch) dieser Komponente oder Komponentengruppe mengenmäßig größer ist als alle anderen jeweils für sich betrachteten Anteile anderer Komponenten oder Komponentengruppen in dem Stoffstrom. Insbesondere bei binären Gemischen ist darunter ein Anteil von mehr als 50 % zu verstehen. Sofern im konkreten Fall nichts anderes angegeben ist, wird hierbei der Massenanteil (Massenbruch) zugrunde gelegt.

Erfindungsgemäß wird die bei dem MTP-Verfahren gemäß Stand der Technik erhaltene Benzinfraktion ("MTP-Benzin") einem Aromatisierungsreaktor zugegeben. Der Aromatisierungsreaktor ist in einem Beispiel als Festbettreaktor aufgebaut und mit einer Schüttung eines festen, partikulären Aromatisierungskatalysators gefüllt. In einem Beispiel wird der Aromatisierungsreaktor im geraden Durchgang ohne Recycle eines Produkt-Teilstroms betrieben.

Erfindungsgemäß wird der Produktstrom des Aromatisierungsreaktors gekühlt und teilkondensiert und sodann in eine Phasentrennvorrichtung eingeleitet. Aus der Phasentrennvorrichtung wird ein gasförmiger Stoffstrom G2 ausgeleitet, der in einem Beispiel ganz oder teilweise dem Verdichter zugeführt wird. Daneben wird ein flüssiger Stoffstroms A erhalten, aus dem durch Aufarbeitung in mehreren Schritten mittels thermischer Trennverfahren letztlich ein Produktstrom gewonnen wird, der BTX-Aromaten enthält.

Dabei wird zunächst der flüssige Stoffstrom A einer Entpentaner-Kolonne zugeführt, in der er in einen Entpentaner-Kopfproduktstrom, der C5- Kohlenwasserstoffe umfasst, und in einen Entpentaner-Sumpfproduktstrom, der C6+ Kohlenwasserstoffe umfasst, aufgetrennt wird.

Der Entpentaner-Sumpfproduktstrom wird einer Aromaten-Trennvorichtung zugeführt, die nach mindestens einem thermischen Trennverfahren, beispielsweise der Destillation oder Extraktivdestillation oder Kombinationen davon arbeitet und in einem Beispiel mehrere hintereinander geschaltete Trennkolonnen umfasst. Als Produkte der Aromaten-Trennvorichtung werden ein erster Produktstrom, der nichtaromatische Kohlenwasserstoff enthält, und ein zweiter Produktstrom erhalten, der BTX-Aromaten enthält. Dieser wird als BTX-Aromaten-Produktstrom aus dem Verfahren ausgeleitet. Je nach der spezifischen Art der verwendeten Trennverfahren können der erste und/oder der zweite Produktstrom jeweils als Kopfproduktstrom, Sumpfproduktstrom oder Seitenstrom der entsprechenden Trennkolonnen erhalten werden.

Von besonderem Vorteil ist es dabei, dass mit der Erfindung BTX-Aromaten als zusätzliche Wertstoffe in guter Ausbeute gewonnen werden, ohne dass sich die Ausbeute an den primären Zielprodukten Ethylen und Propylen verringert, da die BTX-Aromaten durch Weiterverarbeitung der Benzinfraktion des MTP-Verfahrens gemäß Stand der Technik gewonnen werden. Tendenziell erhöht sich dabei die Ausbeute an den primären Zielprodukten Ethylen und Propylen sogar leicht, da durch Behandlung der Benzinfraktion im Aromatisierungsreaktor als Nebenprodukte Komponenten erzeugt werden, die dem Pool der primären Zielprodukte zugeführt werden können.

Zusätzlich verbessert sich mit der Erfindung die Verwendbarkeit der Benzinfraktion: Aufgrund ihres hohen Gehalts an höheren bzw. längerkettigen Olefinen und an Cycloalkanen und Cycloalkenen ist die bei dem MTP-Verfahren gemäß Stand der Technik erhaltene Benzinfraktion nicht ohne Weiteres als Ottokraftstoff einsetzbar. Mit der erfindungsgemäßen Behandlung der Benzinfraktion im Aromatisierungsreaktor wird aber die Konzentration dieser problematischen Komponenten deutlich reduziert, da diese größtenteils zu Aromaten umgewandelt werden. Nach Abtrennen der Aromaten verbleibt eine konvertierte Benzinfraktion, die unproblematisch dem Kohlenwasserstoffpool für die Herstellung von Ottokraftstoffen zugegeben werden kann.

### Weitere Aspekte der Erfindung

Ein zweiter Aspekt der Erfindung ist dadurch gekennzeichnet, dass bei dem Verfahren mindestens ein Teil des gasförmigen Stoffstroms G2 in den Verdichter eingeleitet wird. Da in dem gasförmigen Stoffstrom G2 noch Reaktivkomponenten wie Olefine vorhanden sind, kann auf diese Weise die Ausbeute an den kurzkettigen Olefinen Ethylen und Propylen weiter gesteigert werden.

Ein dritter Aspekt der Erfindung ist dadurch gekennzeichnet, dass bei dem Verfahren mindestens ein Teil des Entpentaner-Kopfproduktstroms zu dem Verdichter zurückgeführt und in diesen eingeleitet wird. Da in dem Entpentaner-Kopfproduktstrom noch Reaktivkomponenten wie Olefine vorhanden sind, kann auf diese Weise die Ausbeute an den kurzkettigen Olefinen Ethylen und Propylen weiter gesteigert werden.

Ein vierter Aspekt der Erfindung ist dadurch gekennzeichnet, dass bei dem Verfahren der zum Verdichter zurückgeführte Teil des Entpentaner-Kopfproduktstroms vor dem Einleiten in den Verdichter mit dem Reaktorproduktstrom vermischt werden. Auf diese Weise wird ein einheitlicherer Einsatzstoffstrom für den Verdichter erhalten und es werden Konzentrationsfluktuationen vermieden. Dies ist insbesondere bei instationären Zuständen des Verfahrens, z. B. Anfahrvorgängen, Abfahrvorgängen oder Lastwechseln von Bedeutung.

Ein fünfter Aspekt der Erfindung ist dadurch gekennzeichnet, dass bei dem Verfahren dass die Aromatisierungsbedingungen eine Reaktortemperatur zwischen 380 und 510 °C, einen Reaktordruck zwischen 1 und 6 bara und die Anwesenheit eines festen Aromatisierungskatalysators umfassen. Untersuchungen haben gezeigt, dass diese Aromatisierungsbedingungen zur Erzielung einer hohen Ausbeute an BTX-Aromaten besonders geeignet sind.

Ein sechster Aspekt der Erfindung ist dadurch gekennzeichnet, dass bei dem Verfahren der Aromatisierungskatalysator in dem Aromatisierungsreaktor als Festbett-Schüttung eines körnigen Katalysators vorliegt, wobei bevorzugt ein Zeolithkatalysator, meist bevorzugt ein Zeolithkatalysator des Strukturtyps ZSM-5 verwendet wird. Aromatisierungskatalysatoren dieses Typs sind bewährt und kommerziell erhältlich.

Ein siebter Aspekt der Erfindung ist dadurch gekennzeichnet, dass bei dem Verfahren als Aromatisierungskatalysator der OTO-Katalysator verwendet wird. Auf diese Weise werden technische und logistische Vorteile erhalten, da sich beispielsweise die Bevorratung frischen oder gebrauchten Katalysators vereinfacht.

Ein achter Aspekt der Erfindung ist dadurch gekennzeichnet, dass bei dem Verfahren als Aromatisierungskatalysator eine Charge des OTO-Katalysators verwendet wird, die zuvor bereits im OTO-Reaktor verwendet wurde. Untersuchungen zeigen, das der bereits im OTO-Reaktor verwendete Katalysator immer noch eine ausreichende Aktivität als Aromatisierungskatalysator aufweist. Es werden daher Ressourcen geschont und ökonomische Vorteile erhalten.

Ein neunter Aspekt der Erfindung ist dadurch gekennzeichnet, dass bei dem Verfahren als Aromatisierungskatalysator eine Charge des OTO-Katalysators verwendet wird, die zuvor bereits während mehrerer Zyklen mit zwischengeschalteter Regenerierung im OTO-Reaktor verwendet wurde. Die Schonung von Ressourcen und die ökonomischen Vorteile verstärken sich hierbei nochmals.

Ein elfter Aspekt der Erfindung ist dadurch gekennzeichnet, dass bei der Anlage ferner Mittel umfasst werden, die es gestatten, dass mindestens ein Teil des gasförmigen Stoffstroms G2 in den Verdichter eingeleitet wird. Die mit diesem Aspekt verbundenen Vorteile entsprechen denen, die im Zusammenhang mit dem zweiten Aspekt der Erfindung erläutert wurden.

Ein zwölfter Aspekt der Erfindung ist dadurch gekennzeichnet, dass bei der Anlage ferner Mittel umfasst werden, die es gestatten, dass mindestens ein Teil des Entpentaner-Kopfproduktstroms zu dem Verdichter zurückgeführt und in diesen eingeleitet wird. Die mit diesem Aspekt verbundenen Vorteile entsprechen denen, die im Zusammenhang mit dem dritten Aspekt der Erfindung erläutert wurden.

Ein dreizehnter Aspekt der Erfindung ist dadurch gekennzeichnet, dass bei der Anlage der Aromatisierungskatalysator in dem Aromatisierungsreaktor als Festbett-Schüttung eines körnigen Katalysator vorliegt, wobei bevorzugt ein Zeolithkatalysator, meist bevorzugt ein Zeolithkatalysator des Strukturtyps ZSM-5 verwendet wird. Die mit diesem Aspekt verbundenen Vorteile entsprechen denen, die im Zusammenhang mit dem sechsten Aspekt der Erfindung erläutert wurden.

Ein vierzehnter Aspekt der Erfindung ist dadurch gekennzeichnet, dass bei der Anlage als Aromatisierungskatalysator der OTO-Katalysator verwendet wird. Die mit diesem Aspekt verbundenen Vorteile entsprechen denen, die im Zusammenhang mit dem siebten Aspekt der Erfindung erläutert wurden.

Ein fünfzehnter Aspekt der Erfindung ist dadurch gekennzeichnet, dass bei der Anlage als Aromatisierungskatalysator eine Charge des OTO-Katalysators verwendet wird, die zuvor bereits im OTO-Reaktor verwendet wurde. Die mit diesem Aspekt verbundenen Vorteile entsprechen denen, die im Zusammenhang mit dem achten Aspekt der Erfindung erläutert wurden.

Ein sechzehnter Aspekt der Erfindung ist dadurch gekennzeichnet, dass bei der Anlage dass als Aromatisierungskatalysator eine Charge des OTO-Katalysators verwendet wird, die zuvor bereits während mehrerer Zyklen mit zwischengeschalteter Regenerierung im OTO-Reaktor verwendet wurde. Die mit diesem Aspekt verbundenen Vorteile entsprechen denen, die im Zusammenhang mit dem neunten Aspekt der Erfindung erläutert wurden.

### Ausführungsbeispiele

Weitere Merkmale, Vorteile und Anwendungsmöglichkeiten der Erfindung ergeben sich aus der nachfolgenden Beschreibung von Ausführungs- und Zahlenbeispielen und der Zeichnungen. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbezügen.

Es zeigen:
- Fig. 1: eine schematische Darstellung eines Verfahrens bzw. einer Anlage gemäß des Stands der Technik,
- Fig. 2: eine schematische Darstellung eines Verfahrens bzw. einer Anlage gemäß der Erfindung.

Im Folgenden ist die Angabe "nicht gezeigt" so zu verstehen, dass ein Element in der besprochenen Abbildung nicht grafisch dargestellt, aber gemäß der Beschreibung trotzdem vorhanden ist.

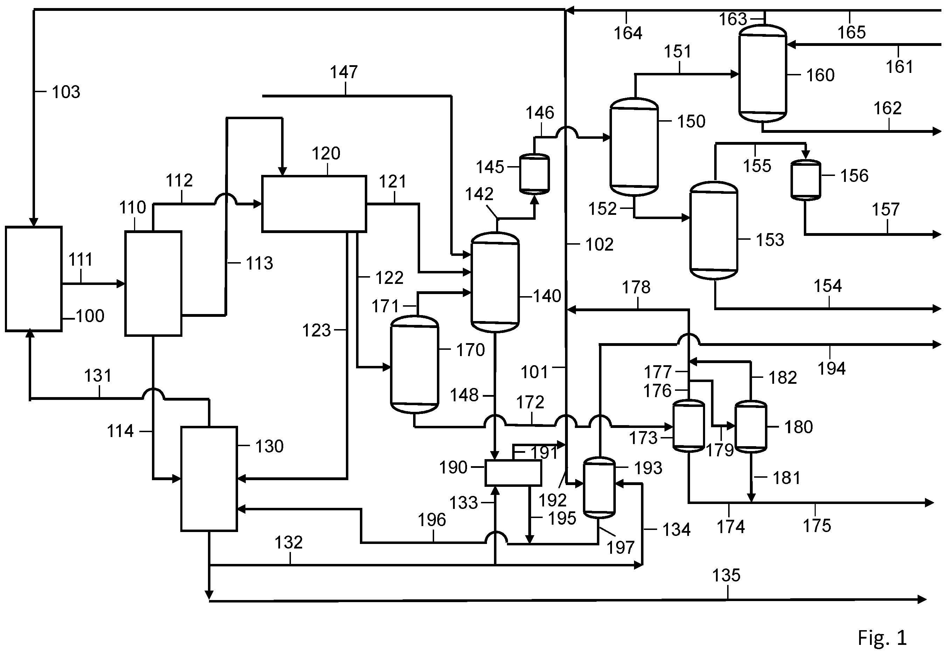

Figur 1 zeigt eine schematische Darstellung eines MTP-Verfahrens bzw. einer MTP-Anlage zur Herstellung von Ethylen und Propylen gemäß des Stands der Technik.

Der OTO-Reaktor 100, in dem die Bildung von Olefinen aus Oxygenaten wie beispielsweise Methanol und/oder Dimethylether (DME) abläuft, wird über eine nicht gezeigte Frischfeed-Leitung mit Oxygenaten sowie Wasserdampf als Verdünnungsmittel beaufschlagt; ferner werden über Leitungen 103 und 131 auch Rückführströme zum Reaktor zurückgeführt. Über Leitung 111 wird der Produktstrom des Reaktors 100 in das Quenchsystem 110 eingebracht. Eine im wesentlichen Wasser enthaltene Phase wird über Leitung 114 aus dem Quenchsystem 110 abgeführt und in eine Methanolrückgewinnungseinheit 130 eingebracht. Die im Quenchsystem 110 abgetrennte gasförmige Phase gelangt über Leitung 112 in einen Verdichter 120. Über Leitung 113 wird zudem eine flüssige, im Wesentlichen aus Kohlenwasserstoffen bestehende Phase aus dem Quenchsystem 110 in den Verdichter 120 eingespeist.

Aus dem Verdichter 120 wird ein flüssiger Strom über Leitung 121 in eine Trennvorrichtung 140 geführt. In dieser Trennvorrichtung 140 wird die C3- Fraktion von der C4 Fraktion getrennt, weshalb sie auch als Depropanizer bezeichnet wird. Die Trennvorrichtung 140 ist vorzugsweise als Trennkolonne ausgestaltet. Vorzugsweise erfolgt die Rektifikation in der Trennvorrichtung 140 als Extraktivdestillation, weshalb über Leitung 147 ein Extraktionsmittel, beispielsweise Methanol, zugeführt werden kann, soweit dies gewünscht ist.

Das über Kopf abgezogene Kopfprodukt, welches die C3- Fraktion enthält, wird über Leitung 142 einem Trockner 145 zugeführt, von dem aus es weiter über Leitung 146 in eine weitere Trennvorrichtung 150 gelangt. Auch eine direkte Verbindung von Trennvorrichtung 140 mit Trennvorrichtung 150 ist möglich. In dieser Trennvorrichtung 150 wird die C3 Fraktion von der C2- Fraktion getrennt, weshalb die Trennvorrichtung 150 auch als Deethanizer bezeichnet wird.

Über Leitung 151 gelangt das Kopfprodukt der Trennvorrichtung 150 in eine Trennvorrichtung 160, welche vorzugsweise als Wäscher ausgebildet ist. Bei einer Ausbildung als Wäscher wird über Leitung 161 ein Waschmittel, beispielsweise Waschwasser eingebracht und über Leitung 162 wieder abgezogen. Der enthaltene C2- Strom wird mit Leitung 163 über Kopf abgezogen und kann über Leitung 164 einem zurück in den Reaktor 100 führenden Recyclestrom in Leitung 103 zugeführt werden und/oder über Leitung 165 aus dem System ausgeleitet und einer weiteren, bildlich nicht gezeigten Trennvorrichtung zur Aufreinigung der enthaltenen Produkte zugeführt werden, um beispielsweise enthaltenes Ethylen als Reinprodukt zu gewinnen.

Das Sumpfprodukt der Trennvorrichtung 150 wird über Leitung 152 einer Trennvorrichtung 153 zugeführt, welche Propan von Propylen trennt. In diesem sogenannten C3 Splitter wird über Leitung 154 Propan aus dem System abgezogen, welches z. B. als Brenngas Verwendung finden kann. Über Leitung 155 wird das Zielprodukt Propylen abgezogen, welches optional in Abhängigkeit von Qualitätsanforderungen einer weiteren Aufreinigungsvorrichtung 156 zugeführt werden kann, aus der es dann über Leitung 157 ausgeleitet wird. Die Aufreinigungsvorrichtung 156 kann beispielsweise mit einem für Oxygenate selektiven Sorbens befüllt werden, so dass Oxygenate aus dem Propylen-Produktstrom je nach Spezifikation bis auf geringe Spuren entfernt werden können.

Über Leitung 122 wird aus dem Verdichter eine flüssige Phase abgezogen, welche einer Trennvorrichtung 170 zugeführt wird, in der die C4- Fraktion und die Oxygenate von der C4+ Fraktion getrennt werden. Diese Trennvorrichtung 170 wird auch als Debutanizer bezeichnet. Über Leitung 171 gelangt die C4- Fraktion und das Oxygenat enthaltende Kopfprodukt der Trennvorrichtung 170 in die Trennvorrichtung 140.

Über Leitung 172 wird das Sumpfprodukt der Trennvorrichtung 170 in eine weitere Trennvorrichtung 173 gegeben, in der die C7+ Fraktion von der C6 Fraktion abgetrennt wird, weshalb die Trennvorrichtung 173 auch als Dehexanizer bezeichnet wird. Das Sumpfprodukt wird über Leitung 174, 175 abgezogen. Über Leitung 176, 177 und 178 kann ein Recyclestrom einer Leitung 101 zugeführt werden, die über Leitungen 102 und 103 zurück zum Reaktor 100 führt.

Aus Leitung 176 kann überdies eine Leitung 179 abgezweigt werden, welche in eine Trennvorrichtung 180 (Benzin-Stabilisatorkolonne) geführt werden. Das hier erhaltene Benzin wird als Wertprodukt über Leitung 181 und Leitung 175 ausgeschleust. Die über Kopf abgetrennten C4 Kohlenwasserstoffe werden über Leitung 182 den Leitungen 177 und 178 zugeführt, so dass sie letztendlich in den Recyclestrom in Leitungen 102, 103 münden.

Das Sumpfprodukt der Trennvorrichtung 140 wird über Leitung 148 abgezogen und gelangt so in eine Mixer-Settler-Kombination 190. Aus dieser werden Kohlenwasserstoffe über Leitung 191 abgezogen und können vollständig oder teilweise über Leitungen 101, 102, 103 in den Reaktor 100 zurückgeführt werden. Alternativ oder ergänzend ist es möglich, die Kohlenwasserstoffe über Leitung 192 einer Trennvorrichtung 193 zuzuführen, welche vorzugsweise als Extraktion ausgestaltet ist. Das Kopfprodukt aus der Trennvorrichtung 193 wird über Leitung 194 als Brenngas ausgeschleust.

Aus der Methanol-Dimethylether-Rückgewinnung 130 wird zum einen ein Oxygenat-Recyclestrom über Leitung 131 in den Reaktor 100 zurückgeführt. Über Leitungen 132 und 133 kann optional mit Methanol und/oder mit Dimethylether beladenes Wasser in die Mixer-Settler-Einheit 190 gespeist werden, aus der es dann auch über die Leitung 195 wieder der Leitung 197 und 196 zugeführt werden kann.

Über Leitungen 132, 134 kann das Wasser als Extraktionsmittel in die Trennvorrichtung 193 geführt werden, sofern diese als Extraktion ausgestaltet ist, aus der es dann auch über die Leitung 197 wieder der Leitung 196 zugeführt werden kann. Über Leitung 135 kann schließlich das Wasser aus dem System ausgeleitet werden.

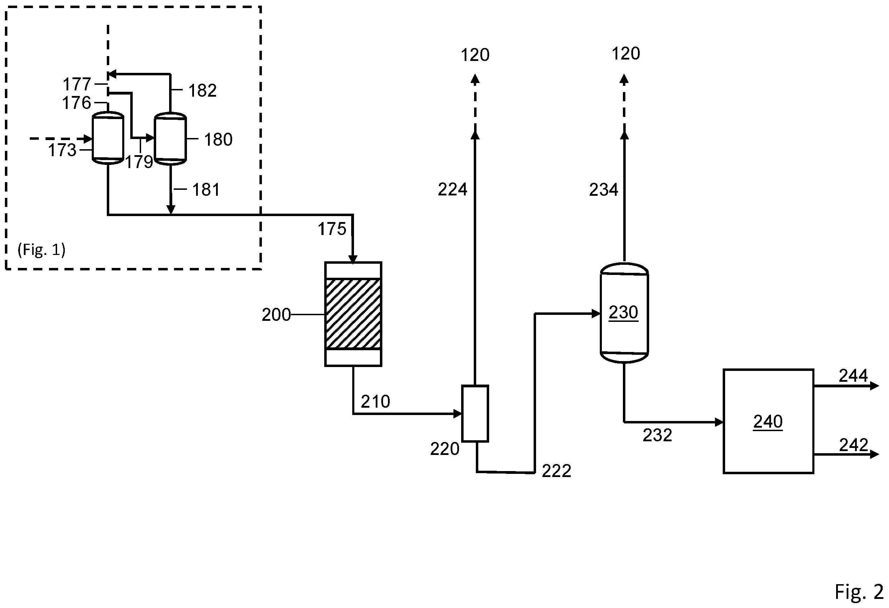

Gemäß Fig. 2 wird erfindungsgemäß der über Leitung 175 ausgeleitete Enthexaner-Sumpfproduktstrom ("MTP-Benzin") nach Vorerhitzung und/oder Verdampfung (nicht gezeigt) einem Aromatisierungsreaktor 200 zugeführt und in diesem unter Aromatisierungsbedingungen zu einem an aromatischen Kohlenwasserstoffen, insbesondere an BTX-Aromaten angereicherten Aromatisierungsreaktor-Produktstrom umgesetzt. In einem Beispiel umfassen die Aromatisierungsbedingungen eine Reaktortemperatur zwischen 380 und 510 °C, einen Reaktordruck zwischen 1 und 6 bara und die Anwesenheit eines festen Aromatisierungskatalysators. In einem Beispiel liegt der Aromatisierungskatalysator in dem Aromatisierungsreaktor als Festbett-Schüttung eines körnigen Katalysators vor, wobei bevorzugt ein Zeolithkatalysator, meist bevorzugt ein Zeolithkatalysator des Strukturtyps ZSM-5 verwendet wird. In einem Beispiel wird als Aromatisierungskatalysator der OTO-Katalysator verwendet. In einem Beispiel wird als Aromatisierungskatalysator eine Charge des OTO-Katalysators verwendet, die zuvor bereits im OTO-Reaktor verwendet wurde. In einem Beispiel wird als Aromatisierungskatalysator eine Charge des OTO-Katalysators verwendet wird, die zuvor bereits während mehrerer Zyklen mit zwischengeschalteter Regenerierung im OTO-Reaktor verwendet wurde.

Der aus dem Aromatisierungsreaktor ausgeleitete, gasförmige Aromatisierungsreaktor-Produktstroms wird abgekühlt und teilkondensiert (beides nicht gezeigt) und sodann über eine Leitung 210 in eine Phasentrennvorrichtung 220 eingeleitet. Aus dieser wird ein gasförmiger Stoffstrom G2 ausgeleitet, der über eine Leitung 224 ausgeleitet wird. In einem Beispiel wird der gasförmige Stoffstrom G2 über Leitung 224 mindestens teilweise zum Verdichter 120 zurückgeführt. Ferner wird aus der Phasentrennvorrichtung 220 ein flüssiger Stoffstrom A über eine Leitung 222 ausgeleitet und in eine Entpentaner-Kolonne 230 eingeleitet.

Aus der Entpentaner-Kolonne 230 wird über eine Leitung 234 ein Entpentaner-Kopfproduktstrom, der C5- Kohlenwasserstoffe umfasst, ausgeleitet. In einem Beispiel wird der Entpentaner-Kopfproduktstrom über Leitung 234 mindestens teilweise zum Verdichter 120 zurückgeführt. Ferner wird über eine Leitung 232 ein Entpentaner-Sumpfproduktstrom, der C6+ Kohlenwasserstoffe umfasst, aus der Entpentaner-Kolonne 230 ausgeleitet und mindestens teilweise in eine nach mindestens einem thermischen Trennverfahren arbeitenden Aromaten-Trennvorrichtung 240 eingeleitet.

In der Aromaten-Trennvorrichtung 240 erfolgt das Auftrennen des Entpentaner-Sumpfproduktstroms in einen ersten Produktstrom, der nichtaromatische Kohlenwasserstoff enthält und über eine Leitung 244 aus dem Verfahren bzw. der Anlage ausgeleitet wird, und in einen zweiten Produktstrom, der BTX-Aromaten enthält und als BTX-Aromaten-Produktstrom über eine Leitung 242 aus dem Verfahren bzw. der Anlage ausgeleitet wird. In einem Beispiel umfasst die Aromaten-Trennvorrichtung 240 mindestens zwei thermische Trennverfahren. In einem Beispiel umfassen die mindestens zwei thermische Trennverfahren die Destillation, Rektifikation, Extraktivdestillation, Extraktion. Die Auswahl und Anwendung dieser thermischen Trennverfahren und die Auswahl geeigneter Betriebsparameter ist dem Fachmann an sich bekannt und/oder kann von ihm durch Routineexperimente ohne Schwierigkeiten ermittelt werden.

### Zahlenbeispiele

Es wurde ein MTP-Benzinstrom von 540 Tagestonnen (tpd) gemäß der Erfindung aufgearbeitet. Die dabei erhaltenen Ergebnisse wurde in den folgenden Tabellen zusammengestellt.

**Tabelle 1: Zusammensetzung des MTP-Benzins (Vergleich)**

| Vergleich (Fig. 1) | Olefine | Paraffine | Naphthene | Aromaten |
|---|---|---|---|---|
| wt-% | 22,3 | 32,5 | 19,0 | 26,2 |
| tpd | 120,42 | 175,5 | 102,6 | 141,48 |

**Tabelle 2: Zusammensetzung des MTP-Benzins nach Aufarbeitung gemäß Erfindung**

| Erfindung (Fig. 2) | Olefine | Paraffine | Naphthene | Aromaten |
|---|---|---|---|---|
| wt-% | 8,6 | 39,1 | 8,6 | 43,7 |
| tpd | 46,13 | 211,21 | 46,55 | 236,12 |

**Tabelle 3: Zusammensetzung des MTP-Benzins (Vergleich): Olefine**

| **Vergleich** | **Ethylen** | **Propylen** | **Butylen** |
|---|---|---|---|
| tpd (im Benzin) | 0 | 0 | 2,7 |

**Tabelle 4: Zusammensetzung des MTP-Benzins nach Aufarbeitung gemäß Erfindung: Olefine**

| **Erfindung** | **Ethylen** | **Propylen** | **Butylen** |
|---|---|---|---|
| tpd (im Benzin) | 5,8 | 11,4 | 4,8 |

**Tabelle 5: Zusammensetzung des MTP-Benzins (Vergleich): BTX-Aromaten**

| **Vergleich** | **Benzol** | **Toluol** | **Xylole** |
|---|---|---|---|
| tpd (im Benzin) | 1,1 | 19,4 | 80,7 |

**Tabelle 6: Zusammensetzung des MTP-Benzins nach Aufarbeitung gemäß Erfindung: BTX-Aromaten**

| **Erfindung** | **Benzol** | **Toluol** | **Xylole** |
|---|---|---|---|
| tpd (im Benzin) | 11,6 | 57,4 | 108,3 |

Anhand der in den Tabellen zusammengestellten Daten ist deutlich zu erkennen, dass durch die erfindungsgemäße Aufarbeitung des MTP-Benzins:
- der Gehalt und die Produktmenge an Aromaten deutlich zunimmt,
- der Gehalt an Olefinen, insbesondere längerkettigen Olefinen, und an Naphthenen (Cycloalkanen) im MTP-Benzins abnimmt,
- der Gehalt und die Produktmenge an kurzkettigen Olefinen, insbesondere an Ethylen und Propylen zunimmt,
- der Gehalt und die Produktmenge an BTX-Aromaten deutlich zunimmt.

### Bezugszeichenliste

| | |
|---|---|
| 100 | OTO-Reaktor |
| 101-103 | Leitung |
| 110 | Quench-System |
| 111-114 | Leitung |
| 120 | Verdichter |
| 121-123 | Leitung |
| 130 | Methanol-DME-Rückgewinnung |
| 131-135 | Leitung |
| 140 | Trennvorrichtung |
| 142 | Leitung |
| 150 | Trennvorrichtung |
| 151, 152 | Leitung |
| 153 | Trennvorrichtung |
| 154, 155 | Leitung |
| 156 | Aufreinigungsstufe |
| 157 | Leitung |
| 160 | Trennvorrichtung |
| 161-165 | Leitung |
| 170 | Trennvorrichtung |
| 171, 172 | Leitung |
| 173 | Trennvorrichtung |
| 174-179 | Leitung |
| 180 | Trennvorrichtung (Benzin-Stabilisatorkolonne) |
| 181, 182 | Leitung |
| 190 | Mixer-Settler-Kombination |
| 191,192 | Leitung |
| 193 | Trennvorrichtung |
| 194-197 | Leitung |
| 200 | Aromatisierungsreaktor |
| 210 | Leitung |
| 220 | Phasentrennvorrichtung |
| 222 | Leitung |
| 224 | Leitung |
| 230 | Entpentaner-Kolonne |
| 232 | Leitung |
| 234 | Leitung |
| 240 | Aromaten-Trennvorrichtung |
| 242 | Leitung |
| 244 | Leitung |

## Patentansprüche

1. Verfahren zur Herstellung von Ethylen, Propylen und BTX-Aromaten aus Oxygenaten, umfassend die folgenden Schritte:
(a) Bereitstellen eines Oxygenate enthaltenden Einsatzstromes und heterogen-katalysiertes Umsetzen der Oxygenate in dem Einsatzstrom in einem Oxygenat-zu-Olefin-Reaktor (OTO-Reaktor) an einem Oxygenat-zu-Olefin-Katalysator (OTO-Katalysator) unter Oxygenatumwandlungsbedingungen zu einem Reaktorproduktstrom, der Wasser, Kohlenwasserstoffe und wenigstens ein Oxygenat enthält;
(b) Quenchen des Reaktorproduktstroms in wenigstens einer Quenchstufe und Verdichten des gequenchten Reaktorproduktstroms in einem der Quenchstufe nachgeschalteten Verdichter, wobei beim Quenchen und/oder Verdichten des Reaktorproduktstroms folgende Stoffströme erhalten werden:
(b1) ein gasförmiger Stoffstrom G1, der Kohlenwasserstoffe umfasst und der spezifisch Olefine umfasst,
(b2) ein flüssiger Stoffstrom W, der Wasser und Oxygenate umfasst,
(b3) ein flüssiger Stoffstrom O, der Kohlenwasserstoffe umfasst und der spezifisch Olefine umfasst;
(c) Mehrstufiges Auftrennen des Stoffstroms G1 mittels thermischer Trennverfahren, wobei eine Gruppe von Produktströmen erhalten wird, wobei die Gruppe von Produktströmen umfasst:
(c1) einen Ethylen enthaltenden Ethylenproduktstrom, der mindestens teilweise aus dem Verfahren ausgeleitet wird,
(c2) einen Propylen enthaltenden Propylenproduktstrom, der aus dem aus dem Verfahren ausgeleitet wird;
(d) Mehrstufiges Auftrennen des Stoffstroms O mittels thermischer Trennverfahren, wobei
(d1) der Stoffstrom O einer Entbutaner-Kolonne zugeführt und in dieser in einen Entbutaner-Kopfproduktstrom und einen Entbutaner-Sumpfproduktstrom aufgetrennt wird, wobei der Entbutaner-Kopfproduktstrom C4- Kohlenwasserstoffe und Oxygenate umfasst und wobei der Entbutaner-Sumpfproduktstrom C4+ Kohlenwasserstoffe umfasst,
(d2) der Entbutaner-Kopfproduktstrom Schritt (c) zugeführt wird,
(d3) der Entbutaner-Sumpfproduktstrom in eine Enthexaner-Kolonne eingeleitet wird;
(e) Auftrennen des Entbutaner-Sumpfproduktstroms in der Enthexaner-Kolonne in einen Enthexaner-Kopfproduktstrom, der C6- Kohlenwasserstoffe umfasst, und in einen Enthexaner-Sumpfproduktstrom, der C7+ Kohlenwasserstoffe umfasst;
(f) Rückführen mindestens eines Teils, bevorzugt des gesamten Enthexaner-Kopfproduktstroms zum OTO-Reaktor;
(g) Verdampfen mindestens eines Teils des Enthexaner-Sumpfproduktstroms und Einleiten des verdampften Enthexaner-Sumpfproduktstroms in einen als Festbett ausgestalteten Aromatisierungsreaktor, Umsetzen des verdampften Enthexaner-Sumpfproduktstroms im Aromatisierungsreaktor unter Aromatisierungsbedingungen, Ausleiten eines gasförmigen Aromatisierungsreaktor-Produktstroms aus dem Aromatisierungsreaktor;
(h) Abkühlen und Teilkondensieren des gasförmigen Aromatisierungsreaktor-Produktstroms und Einleiten des abgekühlten Aromatisierungsreaktor-Produktstroms in eine Phasentrennvorrichtung, Ausleiten eines gasförmigen Stoffstroms G2 und eines flüssigen Stoffstroms A aus der Phasentrennvorrichtung;
(i) Zuführen mindestens eines Teils des flüssigen Stoffstroms A zu einer nach einem thermischen Trennverfahren arbeitenden Entpentaner-Kolonne, Auftrennen des flüssigen Stoffstroms A in der Entpentaner-Kolonne in einen Entpentaner-Kopfproduktstrom, der C5- Kohlenwasserstoffe umfasst, und in einen Entpentaner-Sumpfproduktstrom, der C6+ Kohlenwasserstoffe umfasst;
(j) Zuführen mindestens eines Teils des Entpentaner-Sumpfproduktstroms zu einer nach mindestens einem thermischen Trennverfahren arbeitenden Aromaten-Trennvorrichtung, Auftrennen des Entpentaner-Sumpfproduktstroms in der Aromaten-Trennvorrichtung in einen ersten Produktstrom, der nichtaromatische Kohlenwasserstoff enthält, und in einen zweiten Produktstrom, der BTX-Aromaten enthält und als BTX-Aromaten-Produktstrom aus dem Verfahren ausgeleitet wird.

2. Verfahren nach Anspruch 1, **dadurch gekennzeichnet, dass** mindestens ein Teil des gasförmigen Stoffstroms G2 in den Verdichter eingeleitet wird.

3. Verfahren nach Anspruch 1 oder 2, **dadurch gekennzeichnet, dass** mindestens ein Teil des Entpentaner-Kopfproduktstroms zu dem Verdichter zurückgeführt und in diesen eingeleitet wird.

4. Verfahren nach einem der vorigen Ansprüche, **dadurch gekennzeichnet, dass** der zum Verdichter zurückgeführte Teil des Entpentaner-Kopfproduktstroms vor dem Einleiten in den Verdichter mit dem Reaktorproduktstrom vermischt werden.

5. Verfahren nach einem der vorigen Ansprüche, **dadurch gekennzeichnet, dass** die Aromatisierungsbedingungen eine Reaktortemperatur zwischen 380 und 510 °C, einen Reaktordruck zwischen 1 und 6 bara und die Anwesenheit eines festen Aromatisierungskatalysators umfassen.

6. Verfahren nach Anspruch 5, **dadurch gekennzeichnet, dass** der Aromatisierungskatalysator in dem Aromatisierungsreaktor als Festbett-Schüttung eines körnigen Katalysators vorliegt, wobei bevorzugt ein Zeolithkatalysator, meist bevorzugt ein Zeolithkatalysator des Strukturtyps ZSM-5 verwendet wird.

7. Verfahren nach Anspruch 6, **dadurch gekennzeichnet, dass** als Aromatisierungskatalysator der OTO-Katalysator verwendet wird.

8. Verfahren nach Anspruch 7, **dadurch gekennzeichnet, dass** als Aromatisierungskatalysator eine Charge des OTO-Katalysators verwendet wird, die zuvor bereits im OTO-Reaktor verwendet wurde.

9. Verfahren nach Anspruch 8, **dadurch gekennzeichnet, dass** als Aromatisierungskatalysator eine Charge des OTO-Katalysators verwendet wird, die zuvor bereits während mehrerer Zyklen mit zwischengeschalteter Regenerierung im OTO-Reaktor verwendet wurde.

10. Anlage zur Herstellung von Ethylen, Propylen und BTX-Aromaten aus Oxygenaten, umfassend die folgenden, miteinander in Fluidverbindung stehende Baugruppen und Bestandteile:
(a) einen Oxygenat-zu-Olefin-Reaktor (OTO-Reaktor) mit einem Oxygenat-zu-Olefin-Katalysator (OTO-Katalysator), Mittel zum Bereitstellen eines Oxygenate enthaltenden Einsatzstromes und Mittel zum Einleiten desselben in den OTO-Reaktor, Mittel zum Ausleiten eines Reaktorproduktstroms, der Wasser, Kohlenwasserstoffe und wenigstens ein Oxygenat enthält, aus dem OTO-Reaktor;
(b) eine Quenchstufe und einen der Quenchstufe nachgeschalteten Verdichter, ausgestaltet zum Quenchen des Reaktorproduktstroms in der Quenchstufe und zum Verdichten des gequenchten Reaktorproduktstroms in dem Verdichter, Mittel zum Ausleiten folgender Stoffströme aus der Quenchstufe und/oder aus dem Verdichter:
(b1) ein gasförmiger Stoffstrom G1, der Kohlenwasserstoffe umfasst und der spezifisch Olefine umfasst,
(b2) ein flüssiger Stoffstrom W, der Wasser und Oxygenate umfasst,
(b3) ein flüssiger Stoffstrom O, der Kohlenwasserstoffe umfasst und der spezifisch Olefine umfasst;
(c) eine erste mehrstufige Trennvorrichtung zum Auftrennen des Stoffstroms G1 mittels thermischer Trennverfahren, Mittel zum Ausleiten einer Gruppe von Produktströmen aus der mehrstufige Trennvorrichtung, wobei die Gruppe von Produktströmen umfasst:
(c1) einen Ethylen enthaltenden Ethylenproduktstrom, der mindestens teilweise aus dem Verfahren ausgeleitet wird, wobei ein weiterer Anteil des Ethylenproduktstroms zum OTO-Reaktor zurückgeführt wird,
(c2) einen Propylen enthaltenden Propylenproduktstrom, der aus dem aus dem Verfahren ausgeleitet wird;
(d) eine zweite mehrstufige Trennvorrichtung zum Auftrennen des Stoffstroms O mittels thermischer Trennverfahren, wobei ferner Mittel umfasst werden, die es gestatten, dass
(d1) der Stoffstrom O einer Entbutaner-Kolonne zugeführt und in dieser in einen Entbutaner-Kopfproduktstrom und einen Entbutaner-Sumpfproduktstrom aufgetrennt wird, wobei der Entbutaner-Kopfproduktstrom C4- Kohlenwasserstoffe und Oxygenate umfasst und wobei der Entbutaner-Sumpfproduktstrom C4+ Kohlenwasserstoffe umfasst,
(d2) der Entbutaner-Kopfproduktstrom Schritt (c) zugeführt wird,
(d3) der Entbutaner-Sumpfproduktstrom in eine Enthexaner-Kolonne eingeleitet wird;
(e) Mittel zum Auftrennen des Entbutaner-Sumpfproduktstroms in der Enthexaner-Kolonne in einen Enthexaner-Kopfproduktstrom, der C6- Kohlenwasserstoffe umfasst, und in einen Enthexaner-Sumpfproduktstrom, der C7+ Kohlenwasserstoffe umfasst;
(f) Mittel zum Rückführen mindestens eines Teils, bevorzugt des gesamten Enthexaner-Kopfproduktstroms zum OTO-Reaktor;
(g) Mittel zum Verdampfen mindestens eines Teils des Enthexaner-Sumpfproduktstroms und Mittel zum Einleiten des verdampften Enthexaner-Sumpfproduktstroms in einen als Festbett ausgestalteten Aromatisierungsreaktor, Mittel zum Ausleiten eines gasförmigen Aromatisierungsreaktor-Produktstroms aus dem Aromatisierungsreaktor;
(h) eine Phasentrennvorrichtung, Mittel zum Abkühlen des gasförmigen Aromatisierungsreaktor-Produktstroms und Mittel zum Einleiten des abgekühlten Aromatisierungsreaktor-Produktstroms in die Phasentrennvorrichtung, Mittel zum Ausleiten eines gasförmigen Stoffstroms G2 und eines flüssigen Stoffstroms A aus der Phasentrennvorrichtung;
(i) eine nach einem thermischen Trennverfahren arbeitende Entpentaner-Kolonne, Mittel zum Zuführen mindestens eines Teils des flüssigen Stoffstroms A zu der Entpentaner-Kolonne, Mittel zum Ausleiten eines Entpentaner-Kopfproduktstroms, der C5- Kohlenwasserstoffe umfasst, aus der Entpentaner-Kolonne, und Mittel zum Ausleiten eines Entpentaner-Sumpfproduktstroms, der C6+ Kohlenwasserstoffe umfasst, aus der Entpentaner-Kolonne;
(j) eine nach mindestens einem thermischen Trennverfahren arbeitende Aromaten-Trennvorrichtung, Mittel zum Zuführen mindestens eines Teils des Entpentaner-Sumpfproduktstroms zu der Aromaten-Trennvorrichtung, Mittel zum Ausleiten eines ersten Produktstroms, der nichtaromatische Kohlenwasserstoffe enthält, aus der Aromaten-Trennvorrichtung, und Mittel zum Ausleiten eines zweiten Produktstroms, der BTX-Aromaten enthält, aus der Aromaten-Trennvorrichtung, Mittel zum Ausleiten des zweiten Produktstroms der Aromaten-Trennvorrichtung als BTX-Aromaten-Produktstrom aus der Anlage.

11. Anlage nach Anspruch 10, **dadurch gekennzeichnet, dass** ferner Mittel umfasst werden, die es gestatten, dass mindestens ein Teil des gasförmigen Stoffstroms G2 in den Verdichter eingeleitet wird.

12. Anlage nach Anspruch 10 oder 11, **dadurch gekennzeichnet, dass** ferner Mittel umfasst werden, die es gestatten, dass mindestens ein Teil des Entpentaner-Kopfproduktstroms zu dem Verdichter zurückgeführt und in diesen eingeleitet wird.

13. Anlage nach einem der Ansprüche 10 bis 12, **dadurch gekennzeichnet, dass** der Aromatisierungskatalysator in dem Aromatisierungsreaktor als Festbett-Schüttung eines körnigen Katalysator vorliegt, wobei bevorzugt ein Zeolithkatalysator, meist bevorzugt ein Zeolithkatalysator des Strukturtyps ZSM-5 verwendet wird.

14. Anlage nach einem der Ansprüche 10 bis 13, **dadurch gekennzeichnet, dass** als Aromatisierungskatalysator der OTO-Katalysator verwendet wird.

15. Anlage nach einem der Ansprüche 10 bis 14, **dadurch gekennzeichnet, dass** als Aromatisierungskatalysator eine Charge des OTO-Katalysators verwendet wird, die zuvor bereits im OTO-Reaktor verwendet wurde.

16. Anlage nach einem der Ansprüche 10 bis 15, **dadurch gekennzeichnet, dass** als Aromatisierungskatalysator eine Charge des OTO-Katalysators verwendet wird, die zuvor bereits während mehrerer Zyklen mit zwischengeschalteter Regenerierung im OTO-Reaktor verwendet wurde.
